# EUROPEAN PATENT SPECIFICATION

(11) **EP 3 860 590 B1**
(45) Date of publication and mention of the grant of the patent: **07.06.2023**
(21) Application number: 19790404.8
(22) Date of filing: 03.10.2019
(51) Int. Cl.: A61K 31/404, A61P 37/00

(54) **ETRASIMOD FOR USE IN METHODS FOR THE TREATMENT OF SCLERODERMA**
ETRASIMOD ZUR VERWENDUNG IN METHODEN ZUR BEHANDLUNG VON SKLERODERMIE
ETRASIMOD POUR L'UTILISATION DANS LES MÉTHODES POUR LE TRAITMENT DE LA SKLÉRODERMIE

(30) Priority: 03.10.2018 US 201862740855 P
(43) Date of publication of application: 11.08.2021
(73) Proprietor: Arena Pharmaceuticals, Inc., New York, NY 10001-2192 (US)
(72) Inventor: ADAMS, John W., San Diego, California 92109 (US)
(74) Representative: Pfizer
(86) International application number: PCT/US2019/054576
(87) International publication number: WO 2020/072824

(56) References cited:
- EP-A1- 1 195 165
- WO-A1-2016/209809
- MONIQUE HINCHCLIFF ET AL: "Systemic Sclerosis/Scleroderma: A Treatable Multisystem Disease", AMERICAN FAMILY PHYSICIAN, vol. 78, no. 8, 1 October 2008 (2008-10-01), pages 961-968, XP055346808,
- MIGUEL GUERRERO ET AL: "Sphingosine 1-phosphate receptor 1 agonists: a patent review (2013-2015)", EXPERT OPINION ON THERAPEUTIC PATENTS, vol. 26, no. 4, 15 March 2016 (2016-03-15) , pages 455-470, XP055376536, ISSN: 1354-3776, DOI: 10.1517/13543776.2016.1157165
- DANIEL J. BUZARD ET AL: "Discovery of APD334: Design of a Clinical Stage Functional Antagonist of the Sphingosine-1-phosphate-1 Receptor", ACS MEDICINAL CHEMISTRY LETTERS, vol. 5, no. 12, 4 November 2014 (2014-11-04), pages 1313-1317, XP055477092, US ISSN: 1948-5875, DOI: 10.1021/ml500389m
- CROSBY C M ET AL: "030 Etrasimod, an oral, selective sphingosine 1-phosphate receptor modulator improves skin inflammation in a contact hypersensitivity dermatitis model", JOURNAL OF INVESTIGATIVE DERMATOLOGY; ESDR 2019 ANNUAL MEETING - 49TH ANNUAL ESDR MEETING, ELSEVIER, NL; BORDEAUX, FRANCE, vol. 139, no. 9, Supplement, 1 September 2019 (2019-09-01), page S219, XP009517577, ISSN: 0022-202X, DOI: 10.1016/J.JID.2019.07.033 [retrieved on 2019-09-04]

## Description

### FIELD OF THE INVENTION

The present invention relates to, *inter alia,* (*R*)-2-(7-(4-cyclopentyl-3-(trifluoromethyl)benzyloxy)-1,2,3,4-tetrahydrocyclopenta[b]indol-3-yl)acetic acid **(Compound 1)** for use in methods of treating and/or preventing a condition in an individual in need thereof, wherein the condition is selected from a number of indications including, for example, systemic sclerosis, scleroderma, and CREST syndrome.

### BACKGROUND

The sphingosine-1-phosphate (S1P) receptors 1-5 constitute a family of G protein-coupled receptors with a seven-transmembrane domain. These receptors, referred to as S1P₁ to S1P₅ (formerly termed endothelial differentiation gene (EDG) receptor-1, -5, -3, -6, and -8, respectively; Chun et. al., Pharmacological Reviews, 54:265-269, 2002), are activated *via* binding by sphingosine-1-phosphate, which is produced by the sphingosine kinase-catalyzed phosphorylation of sphingosine. S1P₁, S1P₄, and S1P₅ receptors activate Gi but not Gq, whereas S1P₂ and S1P₃ receptors activate both Gi and Gq. The S1P₃ receptor, but not the S1P₁ receptor, responds to an agonist with an increase in intracellular calcium.

The compound (*R*)-2-(7-(4-cyclopentyl-3-(trifluoromethyl)benzyloxy)-1,2,3,4-tetrahydrocyclopenta[b]indol-3-yl)acetic acid **(Compound 1)** is a potent (EC₅₀ cAMP, 0.093 nM (human)) and selective (EC₅₀ β-arrestin, 6.10 nM (S1P₁), >10,000 nM (S1P₂), >10,000 nM, (S1P₃), 147 nM (S1P₄), and 24.4 nM (S1P₅)), orally available investigational drug candidate for the S1P₁ receptor.

S1P is a signaling sphingolipid required by lymphocytes to exit the lymphoid tissue and enter the bloodstream via a chemotactic gradient. The S1P1 receptor is a physiological mediator which has been shown to regulate lymphocyte recirculation between lymphoid tissue and blood. Binding and internalization of the SIPI receptor may result in lymphocyte retention within lymphoid tissue, with subsequent reduction in peripheral lymphocyte count and lymphocyte availability for recruitment to sites of inflammation. S1P1 receptor surface expression is required for S1P gradient-mediated lymphocyte migration out of lymphoid tissue into the circulation (Brinkmann V., Nat Rev Drug Discov 2010 November; 9(11):883-97).

**Compound 1** is an orally available, selective, sphingosine 1-phosphate receptor (S1P) agonist. The S1P1 receptor is a physiological mediator which has been shown to regulate lymphocyte recirculation between lymphoid tissue and blood. Binding and internalization of the S1P1 receptor may result in lymphocyte retention within lymphoid tissue, with subsequent reduction in peripheral lymphocyte count and lymphocyte availability for recruitment to sites of inflammation. S 1P 1 receptor surface expression is required for S1P gradient-mediated lymphocyte migration out of lymphoid tissue into the circulation (Brinkmann V., et. al., Nat Rev Drug Discov 2010 November; 9 (11):883-97).

A single ascending dose study and a multiple ascending dose study, conducted in healthy subjects, have demonstrated the lymphocyte lowering capabilities of **Compound 1.**

As described herein, (*R*)-2-(7-(4-cyclopentyl-3-(trifluoromethyl)benzyloxy)-1,2,3,4-tetrahydrocyclopenta[b]indol-3-yl)acetic acid **(Compound 1),** or a pharmaceutically acceptable salt, hydrate, or solvate thereof, would offer a novel therapy for scleroderma.

### SUMMARY OF THE INVENTION

The invention is (R)-2-(7-(4-cyclopentyl-3-(trifluoromethyl)benzyloxy)-1,2,3,4-tetrahydrocyclopenta[b]indol-3-yl)acetic acid (Compound 1), or a pharmaceutically acceptable salt, hydrate, or solvate thereof,
for use in a method of treating and/or preventing a condition in an individual in need thereof,
the method comprising administering to the individual a therapeutically effective amount of the Compound 1, or the pharmaceutically acceptable salt, hydrate, or solvate thereof,
wherein the condition is selected from the following: systemic scleroderma; diffuse systemic scleroderma; limited systemic scleroderma; systemic sclerosis; diffuse systemic sclerosis; limited systemic sclerosis; scleroderma; diffuse scleroderma; limited scleroderma; CREST syndrome; diffuse cutaneous systemic sclerosis; limited cutaneous systemic sclerosis; diffuse cutaneous scleroderma; limited cutaneous scleroderma; localized scleroderma; morphea; linear scleroderma; scleroderma en coup de sabre; sine scleroderma; progressive systemic sclerosis; progressive scleroderma; systemic sclerosis with associated interstitial lung disease; scleroderma with associated interstitial lung disease; systemic sclerosis with alveolitis; scleroderma with alveolitis; alveolitis associated with systemic sclerosis; alveolitis associated with scleroderma; systemic sclerosis with lung fibrosis; scleroderma with lung fibrosis; lung fibrosis associated with systemic sclerosis; lung fibrosis associated with scleroderma; systemic sclerosis with pulmonary fibrosis; scleroderma with pulmonary fibrosis; pulmonary fibrosis associated with systemic sclerosis; pulmonary fibrosis associated with scleroderma; renal crisis associated with systemic sclerosis; renal crisis associated with scleroderma; diffuse scleroderma with evidence of fibrosing alveolitis; and systemic sclerosis with diffuse cutaneous involvement.

In one embodiment, the condition is systemic sclerosis.

In one embodiment, the condition is scleroderma.

In one embodiment, the condition is CREST syndrome.

In one embodiment, the individual does not have inflammatory bowel disease or irritable bowel syndrome.

In one embodiment, the individual does not have ulcerative colitis.

In one embodiment, the individual does not have Crohn's disease.

In one embodiment, the therapeutically effective amount of the Compound 1, or the pharmaceutically acceptable salt, hydrate, or solvate thereof, is an amount equivalent to about 1 mg to about 5 mg of Compound 1.

In one embodiment, the therapeutically effective amount of the Compound 1, or the pharmaceutically acceptable salt, hydrate, or solvate thereof, is an amount equivalent to about 1 mg to about 2 mg.

In one embodiment, the therapeutically effective amount of the Compound 1, or the pharmaceutically acceptable salt, hydrate, or solvate thereof, is an amount equivalent to about 1 mg of Compound 1.

In one embodiment, the therapeutically effective amount of the Compound 1, or the pharmaceutically acceptable salt, hydrate, or solvate thereof, is an amount equivalent to about 2 mg of Compound 1.

In one embodiment, the therapeutically effective amount of the Compound 1, or the pharmaceutically acceptable salt, hydrate, or solvate thereof, is an amount equivalent to about 3 mg of Compound 1.

In one embodiment, the Compound 1, or the pharmaceutically acceptable salt, hydrate, or solvate thereof, is selected from: Compound 1, a calcium salt of Compound 1, and an L-arginine salt of Compound 1.

In one embodiment, the Compound 1, or the pharmaceutically acceptable salt, hydrate, or solvate thereof, is an L-arginine salt of Compound 1.

In one embodiment, the Compound 1, or the pharmaceutically acceptable salt, hydrate, or solvate thereof, is an anhydrous, non-solvated crystalline form of the L-arginine salt of Compound 1.

In one embodiment, the Compound 1, or the pharmaceutically acceptable salt, hydrate, or solvate thereof, is a crystalline free-plate habit of the non-solvated L-arginine salt of Compound 1.

In one embodiment, the Compound 1, or the pharmaceutically acceptable salt, hydrate, or solvate thereof, is an anhydrous, non-solvated crystalline form of Compound 1.

### BRIEF DESCRIPTION OF THE INVENTION

References to the methods of treatment by therapy or surgery or *in vivo* diagnosis methods in this description are to be interpreted as references to compounds, pharmaceutical compositions and medicaments of the present invention for use in those methods.

Scleroderma (also referred to as systemic sclerosis) is a multisystem autoimmune disease that can result in progressive fibrosis of the skin and/or internal organs. The condition is characterized by excessive collagen deposition. One form of the condition known as CREST syndrome (also referred to as limited scleroderma) can result in the following features: calcinosis, Raynaud's phenomenon, esophageal dysmotility, sclerodactyly, and telangiectasia.

Described herein are, *inter alia,* methods of treating and/or preventing systemic sclerosis in an individual in need thereof comprising administering a therapeutically effective amount of **Compound 1,** or a pharmaceutically acceptable salt, hydrate, or solvate thereof.

Also described herein are, *inter alia,* methods of treating and/or preventing scleroderma in an individual in need thereof comprising administering a therapeutically effective amount of **Compound 1,** or a pharmaceutically acceptable salt, hydrate, or solvate thereof.

Also described herein are, *inter alia,* methods of treating and/or preventing a condition in an individual in need thereof, comprising administering a therapeutically effective amount Compound 1, or a pharmaceutically acceptable salt, hydrate, or solvate thereof, where the condition is selected from the following: systemic scleroderma; diffuse systemic scleroderma; limited systemic scleroderma; systemic sclerosis; diffuse systemic sclerosis; limited systemic sclerosis; scleroderma; diffuse scleroderma; limited scleroderma; CREST syndrome; diffuse cutaneous systemic sclerosis; limited cutaneous systemic sclerosis; diffuse cutaneous scleroderma; limited cutaneous scleroderma; localized scleroderma; morphea; linear scleroderma; scleroderma en coup de sabre; sine scleroderma; progressive systemic sclerosis; progressive scleroderma; systemic sclerosis with associated interstitial lung disease; scleroderma with associated interstitial lung disease; systemic sclerosis with alveolitis; scleroderma with alveolitis; alveolitis associated with systemic sclerosis; alveolitis associated with scleroderma; systemic sclerosis with lung fibrosis; scleroderma with lung fibrosis; lung fibrosis associated with systemic sclerosis; lung fibrosis associated with scleroderma; systemic sclerosis with pulmonary fibrosis; scleroderma with pulmonary fibrosis; pulmonary fibrosis associated with systemic sclerosis; pulmonary fibrosis associated with scleroderma; renal crisis associated with systemic sclerosis; renal crisis associated with scleroderma; diffuse scleroderma with evidence of fibrosing alveolitis; and systemic sclerosis with diffuse cutaneous involvement.

Also described herein are, *inter alia,* methods of treating and/or preventing skin fibrosis in an individual in need thereof comprising administering a therapeutically effective amount of **Compound** 1, or a pharmaceutically acceptable salt, hydrate, or solvate thereof.

Also described herein are, *inter alia,* methods of treating CREST syndrome in an individual in need thereof comprising administering a therapeutically effective amount of **Compound 1,** or a pharmaceutically acceptable salt, hydrate, or solvate thereof.

Also described herein are, *inter alia,* methods of treating scleroderma in an individual in need thereof comprising administering a therapeutically effective amount of **Compound 1,** or a pharmaceutically acceptable salt, hydrate, or solvate thereof.

Also described herein are uses of **Compound 1,** or a pharmaceutically acceptable salt, hydrate, or solvate thereof, in the manufacture of a medicament for the treatment of scleroderma in an individual in need thereof.

These and other aspects will be set forth in greater detail as the patent disclosure proceeds.

### BRIEF DESCRIPTION OF THE DRAWINGS

**Figure 1** shows the scheme for the administration of **Compound 1** (referred to as APD334) in a bleomycin-induced rodent model of skin fibrosis. mpk = mg/kg.
**Figure 2A** shows the effect of **Compound 1** (referred to as 334) on collagen deposition in a bleomycin-induced rodent model of skin fibrosis.
**Figure 2B** shows the effect of **Compound 1** (referred to as 334) on dermal thickness in a rodent model of skin fibrosis.
**Figure 2C** shows the effect of **Compound 1** (referred to as etrasimod) on the dermis in a bleomycin-induced rodent model of skin fibrosis.
**Figure 3A** shows the effect of **Compound 1** (referred to as 334) on dermal fibrosis in a bleomycin-induced rodent model of skin fibrosis.
**Figure 3B** shows the effect of **Compound 1** (referred to as 334) on inflammation in a rodent model of skin fibrosis.
**Figure 3C** shows the effect of **Compound 1** (referred to as 334) on the average epidermal thickness in a bleomycin-induced rodent model of skin fibrosis.

### DETAILED DESCRIPTION OF THE INVENTION

Again, references to the methods of treatment by therapy or surgery or *in vivo* diagnosis methods in this description are to be interpreted as references to compounds, pharmaceutical compositions and medicaments of the present invention for use in those methods.

Described herein are, *inter alia,* methods of treating scleroderma individual in need thereof comprising administering a therapeutically effective amount of (*R*)-2-(7-(4-cyclopentyl-3-(trifluoromethyl)benzyloxy)-1,2,3,4-tetrahydrocyclopenta[b]indol-3-yl)acetic acid **(Compound 1),** or a pharmaceutically acceptable salt, hydrate, or solvate thereof.

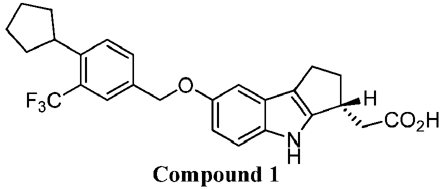

Also described herein are uses of (*R*)-2-(7-(4-cyclopentyl-3-(trifluoromethyl)benzyloxy)-1,2,3,4-tetrahydrocyclopenta[b]indol-3-yl)acetic acid **(Compound 1),** or a pharmaceutically acceptable salt, hydrate, or solvate thereof, in the manufacture of a medicament for the treatment of scleroderma.

Also described herein is (*R*)-2-(7-(4-cyclopentyl-3-(trifluoromethyl)benzyloxy)-1,2,3,4-tetrahydrocyclopenta[b]indol-3-yl)acetic acid **(Compound 1),** or a pharmaceutically acceptable salt, hydrate, or solvate thereof, for use in the treatment of scleroderma.

Also described herein are, *inter alia,* methods of treating scleroderma in an individual in need thereof comprising administering a therapeutically effective amount of (*R*)-2-(7-(4-cyclopentyl-3-(trifluoromethyl)benzyloxy)-1,2,3,4-tetrahydrocyclopenta[b]indol-3-yl)acetic acid **(Compound 1),** or a pharmaceutically acceptable salt, hydrate, or solvate thereof.

Also described herein are uses of (*R*)-2-(7-(4-cyclopentyl-3-(trifluoromethyl)benzyloxy)-1,2,3,4-tetrahydrocyclopenta[b]indol-3-yl)acetic acid **(Compound 1),** or a pharmaceutically acceptable salt, hydrate, or solvate thereof, in the manufacture of a medicament for the treatment of scleroderma in an individual.

Also described herein is (*R*)-2-(7-(4-cyclopentyl-3-(trifluoromethyl)benzyloxy)-1,2,3,4-tetrahydrocyclopenta[b]indol-3-yl)acetic acid **(Compound 1),** or a pharmaceutically acceptable salt, hydrate, or solvate thereof, for use in the treatment of scleroderma.

Certain processes for the preparation of **Compound 1** and the L-arginine salt of **Compound 1** have been previously described (see WO2010/011316 and WO2011/094008). In addition, the novel crystalline plate habit for the L-arginine salt of **Compound 1** has been previously described and is referred to herein as, "crystalline free-plate habit of the non-solvated L-arginine salt of **Compound 1"** (see WO2016/209809).

### CERTAIN EMBODIMENTS

Described herein are, *inter alia,* methods of treating scleroderma in an individual in need thereof comprising administering a therapeutically effective amount of (*R*)-2-(7-(4-cyclopentyl-3-(trifluoromethyl)benzyloxy)-1,2,3,4-tetrahydrocyclopenta[b]indol-3-yl)acetic acid **(Compound 1),** or a pharmaceutically acceptable salt, hydrate, or solvate thereof.

Also described herein are uses of (*R*)-2-(7-(4-cyclopentyl-3-(trifluoromethyl)benzyloxy)-1,2,3,4-tetrahydrocyclopenta[b]indol-3-yl)acetic acid **(Compound 1),** or a pharmaceutically acceptable salt, hydrate, or solvate thereof, in the manufacture of a medicament for the treatment of scleroderma in an individual in need thereof.

One of ordinary skill in the art will understand and appreciate the techniques appropriate for diagnosing and/or evaluating treatment for a condition described herein.

In some embodiments, the condition to be treated and/or prevented is systemic scleroderma. In some embodiments, the condition to be treated and/or prevented is diffuse systemic scleroderma. In some embodiments, the condition to be treated and/or prevented is limited systemic scleroderma. In some embodiments, the condition to be treated and/or prevented is systemic sclerosis. In some embodiments, condition to be treated and/or prevented is diffuse systemic sclerosis. In some embodiments, the condition to be treated and/or prevented is limited systemic sclerosis. In some embodiments, the condition to be treated and/or prevented is scleroderma. In some embodiments, the condition to be treated and/or prevented is diffuse scleroderma. In some embodiments, the condition to be treated and/or prevented is limited scleroderma. In some embodiments, the condition to be treated and/or prevented is CREST syndrome. In some embodiments, the condition to be treated and/or prevented is diffuse cutaneous systemic sclerosis. In some embodiments, the condition to be treated and/or prevented is limited cutaneous systemic sclerosis. In some embodiments, the condition to be treated and/or prevented is diffuse cutaneous scleroderma. In some embodiments, the condition to be treated and/or prevented is limited cutaneous scleroderma. In some embodiments, the condition to be treated and/or prevented is localized scleroderma. In some embodiments, the condition to be treated and/or prevented is morphea. In some embodiments, the condition to be treated and/or prevented is linear scleroderma. In some embodiments, the condition to be treated and/or prevented is scleroderma en coup de sabre. In some embodiments, the condition to be treated and/or prevented is sine scleroderma. In some embodiments, the condition to be treated and/or prevented is progressive. In some embodiments, the condition to be treated and/or prevented is progressive systemic sclerosis. In some embodiments, the condition to be treated and/or prevented is progressive scleroderma. In some embodiments, the condition to be treated and/or prevented is systemic sclerosis with associated interstitial lung disease. In some embodiments, the condition to be treated and/or prevented is scleroderma with associated interstitial lung disease. In some embodiments, the condition to be treated and/or prevented is systemic sclerosis with alveolitis. In some embodiments, the condition to be treated and/or prevented is scleroderma with alveolitis. In some embodiments, the condition to be treated and/or prevented is alveolitis associated with systemic sclerosis. In some embodiments, the condition to be treated and/or prevented is alveolitis associated with scleroderma. In some embodiments, the condition to be treated and/or prevented is systemic sclerosis with lung fibrosis. In some embodiments, the condition to be treated and/or prevented is scleroderma with lung fibrosis. In some embodiments, the condition to be treated and/or prevented is lung fibrosis associated with systemic sclerosis. In some embodiments, the condition to be treated and/or prevented is lung fibrosis associated with scleroderma. In some embodiments, the condition to be treated and/or prevented is systemic sclerosis with pulmonary fibrosis. In some embodiments, the condition to be treated and/or prevented is scleroderma with pulmonary fibrosis. In some embodiments, the condition to be treated and/or prevented is pulmonary fibrosis associated with systemic sclerosis. In some embodiments, the condition to be treated and/or prevented is pulmonary fibrosis associated with scleroderma. In some embodiments, the condition to be treated and/or prevented is renal crisis associated with systemic sclerosis. In some embodiments, the condition to be treated and/or prevented is renal crisis associated with scleroderma. In some embodiments, the condition to be treated and/or prevented is diffuse scleroderma with evidence of fibrosing alveolitis. In some embodiments, the condition to be treated and/or prevented is systemic sclerosis with diffuse cutaneous involvement.

In some embodiments, the condition to be treated and/or prevented is active. In some embodiments, active disease is measured by a worsening skin score. In some embodiments, active disease is measured by a reduction in mRSS points. In some embodiments, active disease is measured by an increase erythrocyte sedimentation rate (ESR). In some embodiments, active disease is detected by new onset of the disease.

In some embodiments, an individual is assessed for inflammation, fibrosis, vasculopathy, and/or autoimmunity. In some embodiments, an individual is assessed for at least one of the following: skin thickening, skin thickening proximal to metacarpophalangeal (MCP) joints, skin thickening of the fingers, puffy fingers, sclerodactyly, fingertip lesions, digital tip ulcers, pitting scars, telangiectasia, abnormal nailfold capillaries, calcinosis, dilated esophagus, scleroderma renal crisis, interstitial lung disease, pulmonary arterial hypertension and/or interstitial lung disease, Raynaud's phenomenon, related antibodies (e.g., an anti-centromere antibody, anti-Scl-70 antibody/antitopoisomerase antibody, anti-fibrillarin autoantibody, anti-RNA polymerase III autoantibody, anti-Th/To, PM-Scl, anti-Ro, anti-U1-ribonucleoprotein, etc.), and collagen mRNA levels in the skin. In some embodiments, the scleroderma is localized to the skin. In some embodiments, the scleroderma involves at least one organ other than the skin. In some embodiments, the scleroderma is referred to as systemic sclerosis. In some embodiments, the scleroderma is CREST syndrome.

In some embodiments, the condition is assessed using a plasma sample. In some embodiments, the condition is assessed using a blood sample. In some embodiments, the condition is assessed using a skin biopsy sample. In some embodiments, the condition is assessed using a fibrotic biomarker. In some embodiments, the condition is assessed using detection of a condition-specific autoantibody.

In some embodiments, the condition is assessed using the modified Rodnan skin score (MRSS). In some embodiments, the MRSS scores for each body site are as follows: 0=no skin involvement; 1=mild thickening; 2=moderate thickening; and 3=severe thickening. In some embodiments, the MRSS at a body site is at least 2. In some embodiments, the individual has moderate skin thickening. In some embodiments, skin thickness is measured in 17 different body sites. In some embodiments, the individual has a total body MRSS score of at least 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or 51.

In some embodiments, the condition is assessed using palpation. In some embodiments, the condition is assessed using palpation on 17 different body areas. In some embodiments, the body areas include at least one of the following: fingers, hands, forearms, arms, feet, legs, and thighs (bilaterally) and face, chest, and abdomen (singly).

In some embodiments, the condition is assessed using the localized scleroderma cutaneous assessment tool (LoSCAT). In some embodiments, the LoSCAT assesses 18 cutaneous anatomic sites. In some embodiments, the LoSCAT captures disease activity (mLoSSI) and damage (LoSDI) parameters. In some embodiments, the LoSCAT takes into consideration cutaneous lesions resulting from localized scleroderma diseases during the inactive stage of the disease. In some embodiments, scores for each site are based on the most severe score for each parameter. In some embodiments, skin changes are compared with the contralateral or ipsilateral skin area to minimize inter-subject variability.

In some embodiments, the Leroy and Medsger criteria is used. In some embodiments, the 1988 Leroy and Medsger criteria is used. In some embodiments, the 2001 Leroy and Medsger criteria is used. In some embodiments, the condition is assessed using the American College of Rheumatology (ACR) criteria. In some embodiments, the condition is assessed using the European League Against Rheumatism (EULAR) criteria. In some embodiments, the 2013 ACR/EULAR diagnostic criteria is used. In some embodiments, an individual is diagnosed with systemic sclerosis if they have a total score of at least 9 using the 2013 ACR/EULAR diagnostic criteria.

In some embodiments, treatment is assessed using the change in a score described herein. In some embodiments, treatment is assessed using the percent change in a measurement described herein. In some embodiments, treatment is assessed using changes in the tele-thermographic profile of cutaneous lesions following treatment. In some embodiments, treatment is assessed using changes in the ultrasound profile of target cutaneous lesion following treatment.

In some embodiments, the condition is assessed using a computed tomography (CT) scan of the lungs. In some embodiments, the condition is assessed by measuring kidney function. In some embodiments, the condition is assessed by measuring blood levels of creatinine. In some embodiments, the condition is assessed using a pulmonary function test. In some embodiments, the condition is assessed using a forced vital capacity (FVC) measure of lung capacity. In some embodiments, the condition is assessed using a diffusing capacity (DLCO) measure of oxygen exchange in the alveoli.

In some embodiments, the condition is assessed using the Dermatology Life Quality Index (DLQI). In some embodiments, the condition is assessed using the Health Assessment Questionnaire-Disability Index (HAQ-DI). In some embodiments, the condition is assessed using the physician global assessment (PGA). In some embodiments, the **Compound 1,** or a pharmaceutically acceptable salt, hydrate, or solvate thereof, is in an amount equivalent to about 1 mg to about 5 mg of

### Compound 1.

In some embodiments, the **Compound 1,** or a pharmaceutically acceptable salt, hydrate, or solvate thereof, is in an amount equivalent to about 3 mg of **Compound 1.**

In some embodiments, the **Compound 1,** or a pharmaceutically acceptable salt, hydrate, or solvate thereof, is in an amount equivalent to about 2.75 mg of **Compound 1.**

In some embodiments, the **Compound 1,** or a pharmaceutically acceptable salt, hydrate, or solvate thereof, is in an amount equivalent to about 2.5 mg of **Compound 1.**

In some embodiments, the **Compound 1,** or a pharmaceutically acceptable salt, hydrate, or solvate thereof, is in an amount equivalent to about 2.25 mg of **Compound 1.**

In some embodiments, the **Compound 1,** or a pharmaceutically acceptable salt, hydrate, or solvate thereof, is in an amount equivalent to about 2 mg of **Compound 1.**

In some embodiments, the **Compound 1,** or a pharmaceutically acceptable salt, hydrate, or solvate thereof, is in an amount equivalent to about 1.5 mg of **Compound 1.**

In some embodiments, the **Compound 1,** or a pharmaceutically acceptable salt, hydrate, or solvate thereof, is in an amount equivalent to about 1 mg of **Compound 1.**

In some embodiments, the **Compound 1,** or a pharmaceutically acceptable salt, hydrate, or solvate thereof, is in an amount equivalent to about 0.5 mg of **Compound 1.**

In some embodiments, the **Compound 1,** or a pharmaceutically acceptable salt, hydrate, or solvate thereof, is selected from: **Compound 1,** a calcium salt of **Compound 1,** and an L-arginine salt of Compound 1.

In some embodiments, the **Compound 1,** or a pharmaceutically acceptable salt, hydrate, or solvate thereof, is an L-arginine salt of Compound 1.

In some embodiments, the **Compound 1,** or a pharmaceutically acceptable salt, hydrate, or solvate thereof, is an anhydrous, non-solvated crystalline form of the L-arginine salt of **Compound 1.**

In some embodiments, the **Compound 1,** or a pharmaceutically acceptable salt, hydrate, or solvate thereof, is a crystalline free-plate habit of the non-solvated L-arginine salt of **Compound 1.**

In some embodiments, the **Compound 1,** or a pharmaceutically acceptable salt, hydrate, or solvate thereof, is an anhydrous, non-solvated crystalline form of **Compound 1.**

In some embodiments, the **Compound 1,** or a pharmaceutically acceptable salt, hydrate, or solvate thereof, is administered orally.

In some embodiments, the **Compound 1,** or a pharmaceutically acceptable salt, hydrate, or solvate thereof, is formulated as a capsule or tablet suitable for oral administration.

In some embodiments, the **Compound 1,** or a pharmaceutically acceptable salt, hydrate, or solvate thereof, is administered once daily.

In some embodiments, the **Compound 1,** or a pharmaceutically acceptable salt, hydrate, or solvate thereof, is administered with food.

In some embodiments, the **Compound 1,** or a pharmaceutically acceptable salt, hydrate, or solvate thereof, is administered without food.

In some embodiments, the individual does not have inflammatory bowel disease.

In some embodiments, the individual does not have irritable bowel syndrome.

In some embodiments, the individual does not have ulcerative colitis.

In some embodiments, the individual does not have Crohn's disease.

In some embodiments, **Compound 1** is administered with food.

In some embodiments, **Compound 1** is administered without food.

In some embodiments, the individual is administered the therapeutically effective amount of **Compound 1** once daily.

### PHARMACEUTICAL COMPOSITIONS

Also described herein are pharmaceutical compositions comprising one or more compounds as described herein and one or more pharmaceutically acceptable carriers. Some embodiments pertain to pharmaceutical compositions comprising a compound as described herein and a pharmaceutically acceptable carrier.

Also described herein is a method of producing a pharmaceutical composition comprising admixing at least one compound as described herein and a pharmaceutically acceptable carrier.

Formulations may be prepared by any suitable method, typically by uniformly mixing the active compound(s) with liquids or finely divided solid carriers, or both, in the required proportions and then, if necessary, forming the resulting mixture into a desired shape.

Conventional excipients, such as binding agents, fillers, acceptable wetting agents, tabletting lubricants and disintegrants may be used in tablets and capsules for oral administration. Liquid preparations for oral administration may be in the form of solutions, emulsions, aqueous or oily suspensions and syrups. Alternatively, the oral preparations may be in the form of dry powder that can be reconstituted with water or another suitable liquid vehicle before use. Additional additives such as suspending or emulsifying agents, non-aqueous vehicles (including edible oils), preservatives and flavorings and colorants may be added to the liquid preparations. Parenteral dosage forms may be prepared by dissolving the compound as described herein in a suitable liquid vehicle and filter sterilizing the solution before filling and sealing an appropriate vial or ampule. These are just a few examples of the many appropriate methods well known in the art for preparing dosage forms.

A compound as described herein can be formulated into pharmaceutical compositions using techniques well known to those in the art. Suitable pharmaceutically acceptable carriers, outside those mentioned herein, are known in the art; for example, see Remington, The Science and Practice of Pharmacy, 20th Edition, 2000, Lippincott Williams & Wilkins, (Editors: Gennaro *et al*.)

While it is possible that, for use in the prophylaxis or treatment, a compound as described herein may, in an alternative use, be administered as a raw or pure chemical, it is preferable however to present the compound or active ingredient as a pharmaceutical formulation or composition further comprising a pharmaceutically acceptable carrier.

Also described herein are pharmaceutical formulations comprising a compound as described herein or a pharmaceutically acceptable salt, solvate, hydrate or derivative thereof together with one or more pharmaceutically acceptable carriers thereof and/or prophylactic ingredients. The carrier(s) must be "acceptable" in the sense of being compatible with the other ingredients of the formulation and not overly deleterious to the recipient thereof.

Pharmaceutical formulations include those suitable for oral, rectal, nasal, topical (including buccal and sub-lingual), vaginal or parenteral (including intramuscular, sub-cutaneous and intravenous) administration or in a form suitable for administration by inhalation, insufflation or by a transdermal patch. Transdermal patches dispense a drug at a controlled rate by presenting the drug for absorption in an efficient manner with a minimum of degradation of the drug. Typically, transdermal patches comprise an impermeable backing layer, a single pressure sensitive adhesive and a removable protective layer with a release liner. One of ordinary skill in the art will understand and appreciate the techniques appropriate for manufacturing a desired efficacious transdermal patch based upon the needs of the artisan.

The compounds as described herein, together with a conventional adjuvant, carrier, or diluent, may thus be placed into the form of pharmaceutical formulations and unit dosages thereof and in such form may be employed as solids, such as tablets or filled capsules, or liquids such as solutions, suspensions, emulsions, elixirs, gels or capsules filled with the same, all for oral use; in the form of suppositories for rectal administration; or in the form of sterile injectable solutions for parenteral (including subcutaneous) use. Such pharmaceutical compositions and unit dosage forms thereof may comprise conventional ingredients in conventional proportions, with or without additional active compounds or principles and such unit dosage forms may contain any suitable effective amount of the active ingredient commensurate with the intended daily dosage range to be employed.

For oral administration, the pharmaceutical composition may be in the form of, for example, a tablet, capsule, suspension or liquid. The pharmaceutical composition is preferably made in the form of a dosage unit containing a particular amount of the active ingredient. Examples of such dosage units are capsules, tablets, powders, granules or suspensions, with conventional additives such as lactose, mannitol, corn starch or potato starch; with binders such as crystalline cellulose, cellulose derivatives, acacia, corn starch or gelatins; with disintegrators such as corn starch, potato starch or sodium carboxymethyl-cellulose; and with lubricants such as talc or magnesium stearate. The active ingredient may also be administered by injection as a composition wherein, for example, saline, dextrose or water may be used as a suitable pharmaceutically acceptable carrier.

Compounds as described herein or a salt, solvate, hydrate or physiologically functional derivative thereof can be used as active ingredients in pharmaceutical compositions, specifically as S1P1 receptor modulators. The term "active ingredient" is defined in the context of a "pharmaceutical composition" and is intended to mean a component of a pharmaceutical composition that provides the primary pharmacological effect, as opposed to an "inactive ingredient" which would generally be recognized as providing no pharmaceutical benefit.

The dose when using the compounds as described herein can vary and as is customary and known to the physician, it is to be tailored to the individual conditions in each individual case. It depends, for example, on the nature and severity of the illness to be treated, on the condition of the individual, on the compound employed or on whether an acute or chronic disease state is treated or prophylaxis is conducted or on whether further active compounds are administered in addition to the compounds as described herein. Representative doses include, but are not limited to, about 1 mg to about 5 mg, about 1 mg, about 1.25 mg, about 1.5 mg, about 1.75 mg, about 2 mg, about 2.25 mg, about 2.5 mg, about 2.75 mg, about 3 mg, about 3.25 mg, about 3.5 mg, about 3.75 mg, about 4 mg, about 4.25 mg, about 4.5 mg, about 4.75 mg, and about 5 mg. Multiple doses may be administered during the day, especially when relatively large amounts are deemed to be needed, for example 2, 3 or 4 doses. Depending on the individual and as deemed appropriate by the individual's physician or caregiver it may be necessary to deviate upward or downward from the doses described herein.

The amount of active ingredient or an active salt, solvate or hydrate derivative thereof, required for use in treatment will vary not only with the particular salt selected but also with the route of administration, the nature of the condition being treated and the age and condition of the individual and will ultimately be at the discretion of the attendant physician or clinician. Representative factors include the type, age, weight, sex, diet and medical condition of the individual, the severity of the disease, the route of administration, pharmacological considerations such as the activity, efficacy, pharmacokinetic and toxicology profiles of the particular compound employed, whether a drug delivery system is utilized, whether an acute or chronic disease state is being treated or prophylaxis is conducted or whether further active compounds are administered in addition to the compounds as described herein and as part of a drug combination. The dosage regimen for treating a disease condition with the compounds and/or compositions as described herein is selected in accordance with a variety factors including those cited above. Thus, the actual dosage regimen employed may vary widely and therefore may deviate from a preferred dosage regimen and one skilled in the art will recognize that dosage and dosage regimens outside these typical ranges can be tested and, where appropriate, may be used in the methods described herein.

The desired dose may conveniently be presented in a single dose or as divided doses administered at appropriate intervals, for example, as 2, 3, 4 or more sub-doses per day. The sub-dose itself may be further divided, *e.g*., into a number of discrete loosely spaced administrations. The daily dose can be divided, especially when relatively large amounts are administered as deemed appropriate, into several, for example 2, 3 or 4-part administrations. If appropriate, depending on individual behavior, it may be necessary to deviate upward or downward from the daily dose indicated.

For preparing pharmaceutical compositions from the compounds as described herein, the suitable pharmaceutically acceptable carrier can be either solid, liquid or a mixture of both. Solid form preparations include powders, tablets, pills, capsules, cachets, suppositories and dispersible granules. A solid carrier can be one or more substances which may also act as diluents, flavoring agents, solubilizers, lubricants, suspending agents, binders, preservatives, tablet disintegrating agents, or encapsulating materials.

In powders, the carrier is a finely divided solid which is in a mixture with the finely divided active component.

In tablets, the active component is mixed with the carrier having the necessary binding capacity in suitable proportions and compacted to the desired shape and size.

The powders and tablets may contain varying percentage amounts of the active compound. A representative amount in a powder or tablet may be from 0.5 to about 90 percent of the active compound. However, an artisan would know when amounts outside of this range are necessary. Suitable carriers for powders and tablets include magnesium carbonate, magnesium stearate, talc, sugar, lactose, pectin, dextrin, starch, gelatin, tragacanth, methylcellulose, sodium carboxymethylcellulose, a low melting wax, cocoa butter and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets and lozenges can be used as solid forms suitable for oral administration.

Liquid form preparations include solutions, suspensions and emulsions, for example, water or water-propylene glycol solutions. For example, parenteral injection liquid preparations can be formulated as solutions in aqueous polyethylene glycol solution. Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. In addition, fatty acids such as oleic acid find use in the preparation of injectables.

The compounds as described herein may thus be formulated for parenteral administration (e.g. by injection, for example bolus injection or continuous infusion) and may be presented in unit dose form in ampoules, pre-filled syringes, small volume infusion or in multi-dose containers with an added preservative. The pharmaceutical compositions may take such forms as suspensions, solutions, or emulsions in oily or aqueous vehicles and may contain formulatory agents such as suspending, stabilizing and/or dispersing agents. Alternatively, the active ingredient may be in powder form, obtained by aseptic isolation of sterile solid or by lyophilization from solution, for constitution with a suitable vehicle, e.g. sterile, pyrogen-free water, before use.

Aqueous formulations suitable for oral use can be prepared by dissolving or suspending the active component in water and adding suitable colorants, flavors, stabilizing and thickening agents, as desired.

Aqueous suspensions suitable for oral use can be made by dispersing the finely divided active component in water with viscous material, such as natural or synthetic gums, resins, methylcellulose, sodium carboxymethylcellulose, or other well-known suspending agents.

Also included are solid form preparations which are intended to be converted, shortly before use, to liquid form preparations for oral administration. Such liquid forms include solutions, suspensions and emulsions. These preparations may contain, in addition to the active component, colorants, flavors, stabilizers, buffers, artificial and natural sweeteners, dispersants, thickeners, solubilizing agents and the like.

For topical administration to the epidermis the compounds as described herein may be formulated as ointments, creams or lotions, or as a transdermal patch.

Ointments and creams may, for example, be formulated with an aqueous or oily base with the addition of suitable thickening and/or gelling agents. Lotions may be formulated with an aqueous or oily base and will in general also contain one or more emulsifying agents, stabilizing agents, dispersing agents, suspending agents, thickening agents, or coloring agents.

Formulations suitable for topical administration in the mouth include lozenges comprising the active agent in a flavored base, usually sucrose and acacia or tragacanth; pastilles comprising the active ingredient in an inert base such as gelatin and glycerin or sucrose and acacia; and mouthwashes comprising the active ingredient in a suitable liquid carrier.

Solutions or suspensions are applied directly to the nasal cavity by conventional means, for example with a dropper, pipette or spray. The formulations may be provided in single or multi-dose form. In the latter case of a dropper or pipette, this may be achieved by the individual administering an appropriate, predetermined volume of the solution or suspension. In the case of a spray, this may be achieved for example by means of a metering atomizing spray pump.

Administration to the respiratory tract may also be achieved by means of an aerosol formulation in which the active ingredient is provided in a pressurized pack with a suitable propellant. If the compounds as described herein or pharmaceutical compositions comprising them are administered as aerosols (e.g., nasal aerosols, by inhalation), this can be carried out, for example, using a spray, a nebulizer, a pump nebulizer, an inhalation apparatus, a metered inhaler or a dry powder inhaler. Pharmaceutical forms for administration of the compounds as described herein as an aerosol can be prepared by processes well known to the person skilled in the art. Solutions or dispersions of the compounds as described herein or a pharmaceutically acceptable salt, solvate, hydrate or derivative thereof in water, water/alcohol mixtures or suitable saline solutions, for example, can be employed using customary additives (*e*.*g*., benzyl alcohol or other suitable preservatives), absorption enhancers for increasing the bioavailability, solubilizers, dispersants and others and, if appropriate, customary propellants (*e*.*g*., carbon dioxide, CFCs, such as, dichlorodifluoromethane, trichlorofluoromethane, dichlorotetrafluoroethane and the like). The aerosol may conveniently also contain a surfactant such as lecithin. The dose of drug may be controlled by provision of a metered valve.

In formulations intended for administration to the respiratory tract, including intranasal formulations, the compound will generally have a small particle size for example of the order of 10 microns or less. Such a particle size may be obtained by means known in the art, for example by micronization. When desired, formulations adapted to give sustained release of the active ingredient may be employed.

Alternatively, the active ingredients may be provided in the form of a dry powder (*e*.*g*., a powder mix of the compound in a suitable powder base such as lactose, starch, starch derivatives such as hydroxypropylmethyl cellulose and polyvinylpyrrolidone (PVP)). Conveniently the powder carrier will form a gel in the nasal cavity. The powder composition may be presented in unit dose form (*e.g*., capsules, cartridges) as for gelatin or blister packs from which the powder may be administered by means of an inhaler.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packeted tablets, capsules and powders in vials or ampoules. Also, the unit dosage form can be a capsule, tablet, cachet, or lozenge itself, or it can be the appropriate number of any of these in packaged form.

In some embodiments, the compositions are tablets or capsules for oral administration.

In some embodiments, the compositions are liquids for intravenous administration.

The compounds as described herein may optionally exist as pharmaceutically acceptable salts including pharmaceutically acceptable acid addition salts prepared from pharmaceutically acceptable non-toxic acids including inorganic and organic acids. Representative acids include, but are not limited to, acetic, benzenesulfonic, benzoic, camphorsulfonic, citric, ethenesulfonic, dichloroacetic, formic, fumaric, gluconic, glutamic, hippuric, hydrobromic, hydrochloric, isethionic, lactic, maleic, malic, mandelic, methanesulfonic, mucic, nitric, oxalic, pamoic, pantothenic, phosphoric, succinic, sulfiric, tartaric, oxalic, p-toluenesulfonic and the like, such as those pharmaceutically acceptable salts listed by Berge et al., Journal of Pharmaceutical Sciences, 66:1-19 (1977).

The acid addition salts may be obtained as the direct products of compound synthesis. In the alternative, the free base may be dissolved in a suitable solvent containing the appropriate acid and the salt isolated by evaporating the solvent or otherwise separating the salt and solvent. The compounds as described herein may form solvates with standard low molecular weight solvents using methods known to the skilled artisan.

When an integer is used in a method disclosed herein, the term "about" can be inserted before the integer.

As will be recognized, the steps of the methods described herein need not be performed any particular number of times or in any particular sequence. Additional objects, advantages and novel features of this invention will become apparent to those skilled in the art upon examination of the following examples thereof, which are intended to be illustrative and not intended to be limiting.

### EXAMPLES

### Example 1: Preparation of Compounds

The preparation of **Compound 1,** including certain crystal forms of **Compound 1** are described in International Patent Application No. PCT/US2009/004265, published as International Publication No. WO2010/011316, and International Patent Application No. PCT/US2011/000153, published as International Publication No. WO2011/094008.

The preparation of the crystalline free-plate habit of the non-solvated L-arginine salt of **Compound 1** is described in International Patent Application No. PCT/US2016/038506, published as International Publication No. WO2016/209809.

### Example 2:

**Compound 1** was evaluated in a bleomycin-induced rodent model of skin fibrosis. Skin fibrosis was induced in male C57BL/6 mice (6-7 weeks old; 20-25 g) with bleomycin injections at a concentration of 0.5 mg/ml via subcutaneous (SC) route. The mice received 100 µl of bleomycin at 0.5 mg/ml dissolved in PBS every other day for 21 days.

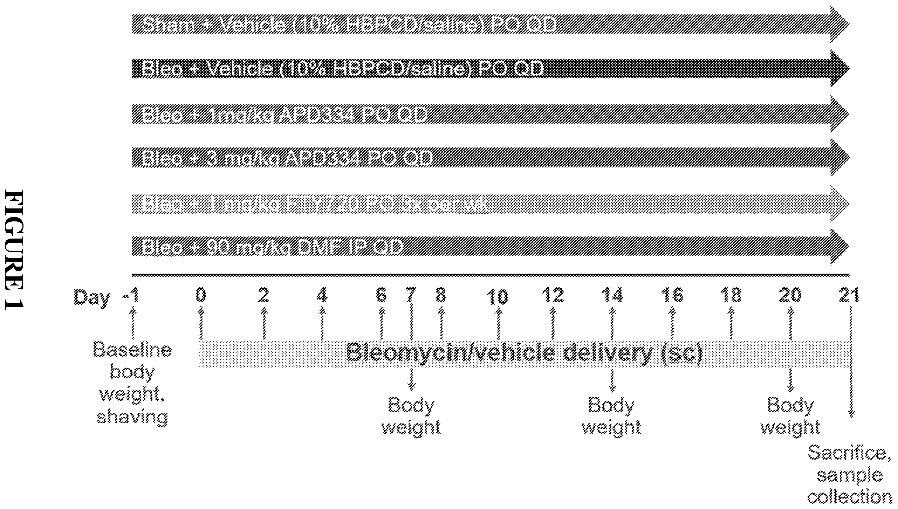

The following treatments were randomly assigned: 1.) sham + vehicle (10% HBPCD/saline) per oral (PO) once daily (QD); 2.) bleomycin + vehicle (10% HBPCD/saline) PO QD; 3.) bleomycin + 1 mg/kg **Compound 1** PO QD; 4.) bleomycin + 3 mg/kg **Compound 1** PO QD; 5.) bleomycin + 1 mg/kg FTY720 PO 3x/week; 6.) bleomycin + 90 mg/kg FTY720 intraperitoneal (IP) QD **(****Figure 1****).**

Skin tissue was harvested post-mortem, with half of the biopsy specimen fixed in 4 % wt/vol. paraformaldehyde for histology and the other half snap frozen for collagen analysis (hydroxyproline assay). Histological sections (4 µm) were cut from paraffin-embedded PFA-fixed lesional skin tissue. Sections were stained with haematoxylin and eosin (H&E) and Masson's Trichrome. Images were captured at × 100 microscopic magnification for histological dermal thickness determination.

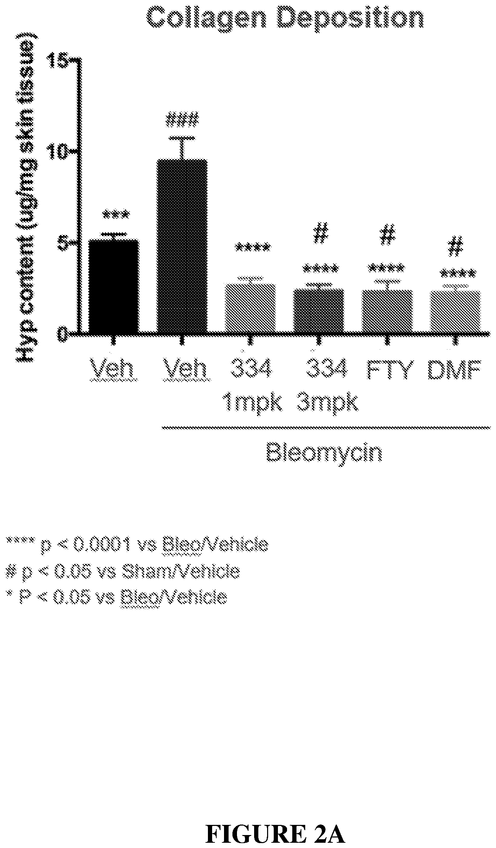

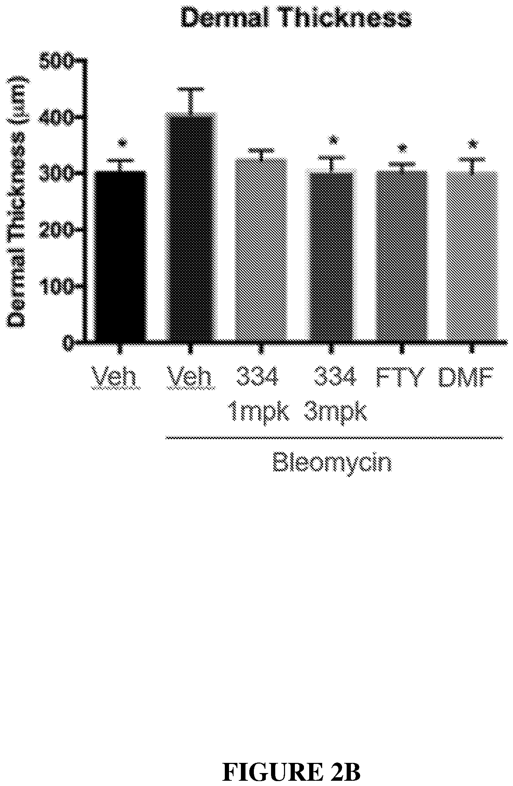

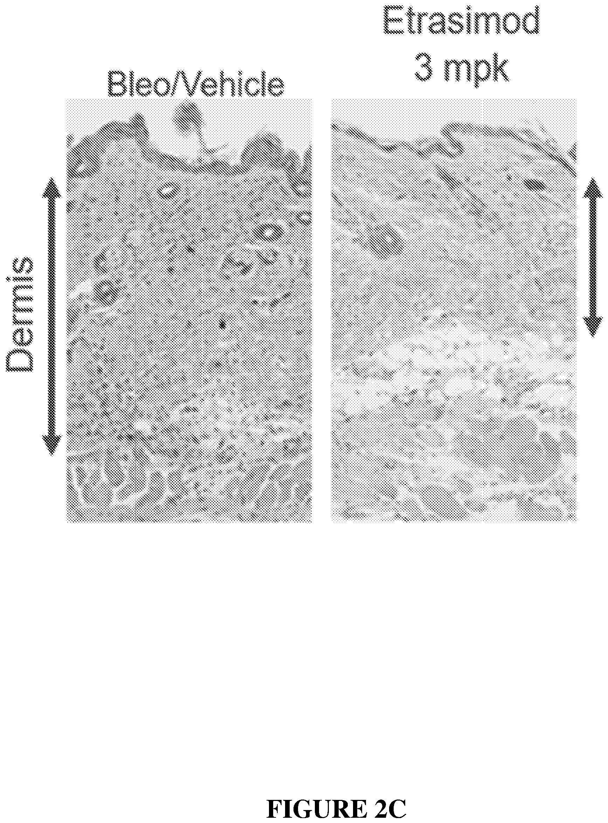

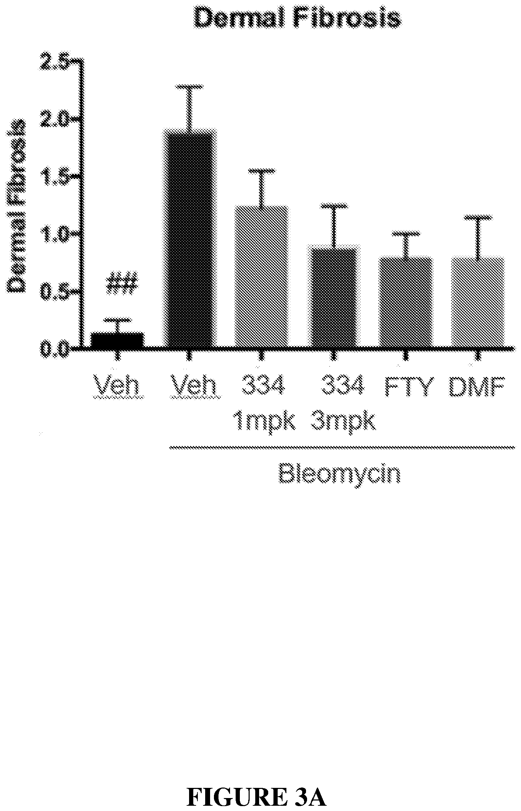

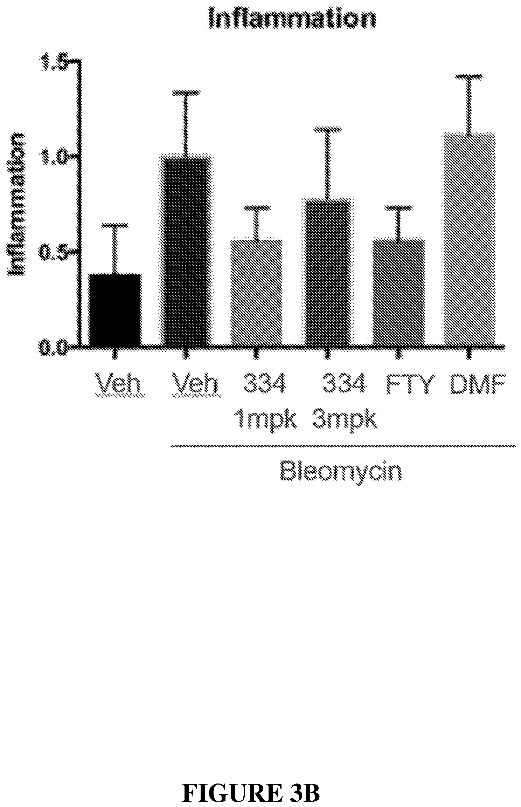

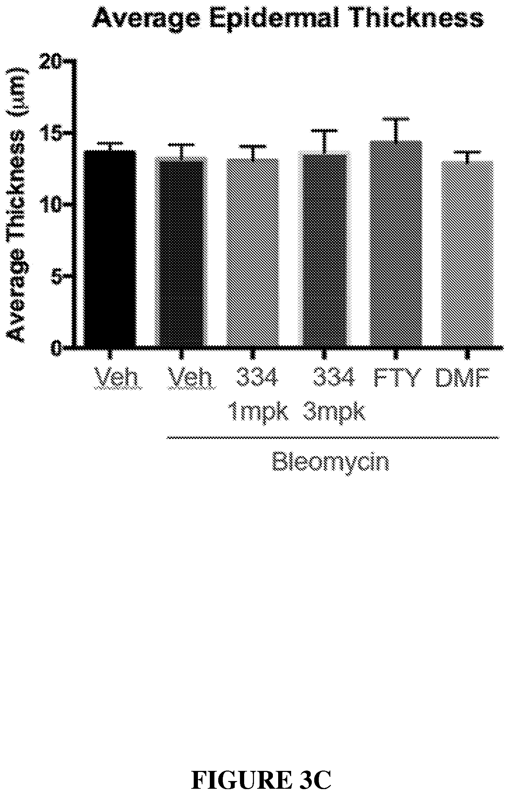

Collagen deposition **(****Figure 2A****),** dermal thickness **(****Figure 2B****),** tissue sections **(****Figure 2C****),** dermal fibrosis **(****Figure 3A****),** inflammation **(****Figure 3B****),** and average epidermal thickness **(****Figure 3C****)** were evaluated.

**Compound 1** significantly reduced collagen deposition **(****Figure 2A****)** and dermal thickening **(****Figure 2B****)** in the bleomycin-induced skin fibrosis model.

## Claims

1. (R)-2-(7-(4-cyclopentyl-3-(trifluoromethyl)benzyloxy)-1,2,3,4-tetrahydrocyclopenta[b]indol-3-yl)acetic acid (Compound 1),
or a pharmaceutically acceptable salt, hydrate, or solvate thereof,
for use in a method of treating and/or preventing a condition in an individual in need thereof,
the method comprising administering to the individual a therapeutically effective amount of the Compound 1, or the pharmaceutically acceptable salt, hydrate, or solvate thereof,
wherein the condition is selected from the following: systemic scleroderma; diffuse systemic scleroderma; limited systemic scleroderma; systemic sclerosis; diffuse systemic sclerosis; limited systemic sclerosis; scleroderma; diffuse scleroderma; limited scleroderma; CREST syndrome; diffuse cutaneous systemic sclerosis; limited cutaneous systemic sclerosis; diffuse cutaneous scleroderma; limited cutaneous scleroderma; localized scleroderma; morphea; linear scleroderma; scleroderma en coup de sabre; sine scleroderma; progressive systemic sclerosis; progressive scleroderma; systemic sclerosis with associated interstitial lung disease; scleroderma with associated interstitial lung disease; systemic sclerosis with alveolitis; scleroderma with alveolitis; alveolitis associated with systemic sclerosis; alveolitis associated with scleroderma; systemic sclerosis with lung fibrosis; scleroderma with lung fibrosis; lung fibrosis associated with systemic sclerosis; lung fibrosis associated with scleroderma; systemic sclerosis with pulmonary fibrosis; scleroderma with pulmonary fibrosis; pulmonary fibrosis associated with systemic sclerosis; pulmonary fibrosis associated with scleroderma; renal crisis associated with systemic sclerosis; renal crisis associated with scleroderma; diffuse scleroderma with evidence of fibrosing alveolitis; and systemic sclerosis with diffuse cutaneous involvement.

2. (R)-2-(7-(4-cyclopentyl-3-(trifluoromethyl)benzyloxy)-1,2,3,4-tetrahydrocyclopenta[b]indol-3-yl)acetic acid (Compound 1),
or a pharmaceutically acceptable salt, hydrate, or solvate thereof,
for use according to claim 1,
wherein the condition is systemic sclerosis.

3. (R)-2-(7-(4-cyclopentyl-3-(trifluoromethyl)benzyloxy)-1,2,3,4-tetrahydrocyclopenta[b]indol-3-yl)acetic acid (Compound 1),
or a pharmaceutically acceptable salt, hydrate, or solvate thereof,
for use according to claim 1,
wherein the condition is scleroderma.

4. (R)-2-(7-(4-cyclopentyl-3-(trifluoromethyl)benzyloxy)-1,2,3,4-tetrahydrocyclopenta[b]indol-3-yl)acetic acid (Compound 1),
or a pharmaceutically acceptable salt, hydrate, or solvate thereof,
for use according to claim 1,
wherein the condition is CREST syndrome.

5. (R)-2-(7-(4-cyclopentyl-3-(trifluoromethyl)benzyloxy)-1,2,3,4-tetrahydrocyclopenta[b]indol-3-yl)acetic acid (Compound 1),
or a pharmaceutically acceptable salt, hydrate, or solvate thereof,
for use according to any one of claims 1-4,
wherein the individual does not have inflammatory bowel disease or irritable bowel syndrome.

6. (R)-2-(7-(4-cyclopentyl-3-(trifluoromethyl)benzyloxy)-1,2,3,4-tetrahydrocyclopenta[b]indol-3-yl)acetic acid (Compound 1),
or a pharmaceutically acceptable salt, hydrate, or solvate thereof,
for use according to any one of claims 1-5,
wherein the individual does not have ulcerative colitis.

7. (R)-2-(7-(4-cyclopentyl-3-(trifluoromethyl)benzyloxy)-1,2,3,4-tetrahydrocyclopenta[b]indol-3-yl)acetic acid (Compound 1),
or a pharmaceutically acceptable salt, hydrate, or solvate thereof,
for use according to any one of claims 1-6,
wherein the individual does not have Crohn's disease.

8. (R)-2-(7-(4-cyclopentyl-3-(trifluoromethyl)benzyloxy)-1,2,3,4-tetrahydrocyclopenta[b]indol-3-yl)acetic acid (Compound 1),
or a pharmaceutically acceptable salt, hydrate, or solvate thereof,
for use according to any one of claims 1-7,
wherein the therapeutically effective amount of the Compound 1, or the pharmaceutically acceptable salt, hydrate, or solvate thereof, is an amount equivalent to about 1 mg to about 5 mg of Compound 1.

9. (R)-2-(7-(4-cyclopentyl-3-(trifluoromethyl)benzyloxy)-1,2,3,4-tetrahydrocyclopenta[b]indol-3-yl)acetic acid (Compound 1),
or a pharmaceutically acceptable salt, hydrate, or solvate thereof,
for use according to any one of claims 1-7,
wherein the therapeutically effective amount of the Compound 1, or the pharmaceutically acceptable salt, hydrate, or solvate thereof, is an amount equivalent to about 1 mg to about 2 mg.

10. (R)-2-(7-(4-cyclopentyl-3-(trifluoromethyl)benzyloxy)-1,2,3,4-tetrahydrocyclopenta[b]indol-3-yl)acetic acid (Compound 1),
or a pharmaceutically acceptable salt, hydrate, or solvate thereof,
for use according to any one of claims 1-7,
wherein the therapeutically effective amount of the Compound 1, or the pharmaceutically acceptable salt, hydrate, or solvate thereof, is an amount equivalent to about 1 mg of Compound 1.

11. (R)-2-(7-(4-cyclopentyl-3-(trifluoromethyl)benzyloxy)-1,2,3,4-tetrahydrocyclopenta[b]indol-3-yl)acetic acid (Compound 1),
or a pharmaceutically acceptable salt, hydrate, or solvate thereof,
for use according to any one of claims 1-7,
wherein the therapeutically effective amount of the Compound 1, or the pharmaceutically acceptable salt, hydrate, or solvate thereof, is an amount equivalent to about 2 mg of Compound 1.

12. (R)-2-(7-(4-cyclopentyl-3-(trifluoromethyl)benzyloxy)-1,2,3,4-tetrahydrocyclopenta[b]indol-3-yl)acetic acid (Compound 1),
or a pharmaceutically acceptable salt, hydrate, or solvate thereof,
for use according to any one of claims 1-7,
wherein the therapeutically effective amount of the Compound 1, or the pharmaceutically acceptable salt, hydrate, or solvate thereof, is an amount equivalent to about 3 mg of Compound 1.

13. (R)-2-(7-(4-cyclopentyl-3-(trifluoromethyl)benzyloxy)-1,2,3,4-tetrahydrocyclopenta[b]indol-3-yl)acetic acid (Compound 1),
or a pharmaceutically acceptable salt, hydrate, or solvate thereof,
for use according to any one of claims 1-12,
wherein the Compound 1, or the pharmaceutically acceptable salt, hydrate, or solvate thereof, is selected from: Compound 1, a calcium salt of Compound 1, and an L-arginine salt of Compound 1.

14. (R)-2-(7-(4-cyclopentyl-3-(trifluoromethyl)benzyloxy)-1,2,3,4-tetrahydrocyclopenta[b]indol-3-yl)acetic acid (Compound 1),
or a pharmaceutically acceptable salt, hydrate, or solvate thereof,
for use according to any one of claims 1-12,
wherein the Compound 1, or the pharmaceutically acceptable salt, hydrate, or solvate thereof, is an L-arginine salt of Compound 1.

15. (R)-2-(7-(4-cyclopentyl-3-(trifluoromethyl)benzyloxy)-1,2,3,4-tetrahydrocyclopenta[b]indol-3-yl)acetic acid (Compound 1),
or a pharmaceutically acceptable salt, hydrate, or solvate thereof,
for use according to any one of claims 1-14,
wherein the Compound 1, or the pharmaceutically acceptable salt, hydrate, or solvate thereof, is an anhydrous, non-solvated crystalline form of the L-arginine salt of Compound 1.

16. (R)-2-(7-(4-cyclopentyl-3-(trifluoromethyl)benzyloxy)-1,2,3,4-tetrahydrocyclopenta[b]indol-3-yl)acetic acid (Compound 1),
or a pharmaceutically acceptable salt, hydrate, or solvate thereof,
for use according to any one of claims 1-14,
wherein the Compound 1, or the pharmaceutically acceptable salt, hydrate, or solvate thereof, is a crystalline free-plate habit of the non-solvated L-arginine salt of Compound 1.

17. (R)-2-(7-(4-cyclopentyl-3-(trifluoromethyl)benzyloxy)-1,2,3,4-tetrahydrocyclopenta[b]indol-3-yl)acetic acid (Compound 1),
or a pharmaceutically acceptable salt, hydrate, or solvate thereof,
for use according to any one of claims 1-12,
wherein the Compound 1, or the pharmaceutically acceptable salt, hydrate, or solvate thereof, is an anhydrous, non-solvated crystalline form of Compound 1.

## Patentansprüche

1. (R)-2-(7-(4-Cyclopentyl-3-(trifluormethyl)benzyloxy)-1,2,3,4-tetrahydrocyclopenta[b]indol-3-yl)essigsäure (Verbindung 1) oder ein pharmazeutisch annehmbares Salz, Hydrat oder Solvat davon zur Verwendung in einem Verfahren zur Behandlung und/oder Vorbeugung eines Zustands bei einem Individuum, das Bedarf daran hat,
wobei das Verfahren die Verabreichung einer therapeutisch wirksamen Menge der Verbindung 1 oder des pharmazeutisch akzeptablen Salzes, Hydrats oder Solvats davon an das Individuum umfasst,
wobei der Zustand aus den folgenden ausgewählt ist: systemische Sklerodermie; diffuse systemische Sklerodermie;; systemische Sklerose; diffuse systemische Sklerose; begrenzte systemische Sklerose; Sklerodermie; diffuse Sklerodermie; begrenzte Sklerodermie; CREST-Syndrom; diffuse kutane systemische Sklerose; begrenzte kutane systemische Sklerose; diffuse kutane Sklerodermie; begrenzte kutane Sklerodermie; lokalisierte Sklerodermie; Morphea; lineare Sklerodermie; Sklerodermie en coup de sabre; sine Sklerodermie; progressive systemische Sklerose; progressive Sklerodermie; systemische Sklerose mit assoziierter interstitieller Lungenerkrankung; Sklerodermie mit assoziierter interstitieller Lungenerkrankung; systemische Sklerose mit Alveolitis; Sklerodermie mit Alveolitis; Alveolitis in Verbindung mit systemischer Sklerose; Alveolitis in Verbindung mit Sklerodermie; systemische Sklerose mit Lungenfibrose; Sklerodermie mit Lungenfibrose; Lungenfibrose in Verbindung mit systemischer Sklerose; Lungenfibrose in Verbindung mit Sklerodermie; systemische Sklerose mit Lungenfibrose; Sklerodermie mit Lungenfibrose; Lungenfibrose in Verbindung mit systemischer Sklerose; Lungenfibrose in Verbindung mit Sklerodermie; Nierenkrise in Verbindung mit systemischer Sklerose; Nierenkrise in Verbindung mit Sklerodermie; diffuse Sklerodermie mit Anzeichen einer fibrosierenden Alveolitis; und systemische Sklerose mit diffuser Hautbeteiligung.

2. (R)-2-(7-(4-Cyclopentyl-3-(trifluormethyl)benzyloxy)-1,2,3,4-tetrahydrocyclopenta[b]indol-3-yl)essigsäure (Verbindung 1) oder ein pharmazeutisch akzeptables Salz, Hydrat oder Solvat davon
zur Verwendung nach Anspruch 1,
wobei es sich um systemische Sklerose handelt.

3. (R)-2-(7-(4-Cyclopentyl-3-(trifluormethyl)benzyloxy)-1,2,3,4-tetrahydrocyclopenta[b]indol-3-yl)essigsäure (Verbindung 1) oder ein pharmazeutisch akzeptables Salz, Hydrat oder Solvat davon
zur Verwendung nach Anspruch 1, wobei die Erkrankung Sklerodermie ist.

4. (R)-2-(7-(4-Cyclopentyl-3-(trifluormethyl)benzyloxy)-1,2,3,4-tetrahydrocyclopenta[b]indol-3-yl)essigsäure (Verbindung 1) oder ein pharmazeutisch akzeptables Salz, Hydrat oder Solvat davon
zur Verwendung nach Anspruch 1, wobei der Zustand das CREST-Syndrom ist.

5. (R)-2-(7-(4-Cyclopentyl-3-(trifluormethyl)benzyloxy)-1,2,3,4-tetrahydrocyclopenta[b]indol-3-yl)essigsäure (Verbindung 1) oder ein pharmazeutisch akzeptables Salz, Hydrat oder Solvat davon
zur Verwendung nach einem der Ansprüche 1-4, wobei die Person keine chronisch-entzündliche Darmerkrankung oder kein Reizdarmsyndrom hat.

6. (R)-2-(7-(4-Cyclopentyl-3-(trifluormethyl)benzyloxy)-1,2,3,4-tetrahydrocyclopenta[b]indol-3-yl)essigsäure (Verbindung 1) oder ein pharmazeutisch akzeptables Salz, Hydrat oder Solvat davon
zur Verwendung nach einem der Ansprüche 1-5, wobei die Person keine Colitis ulcerosa hat.

7. (R)-2-(7-(4-Cyclopentyl-3-(trifluormethyl)benzyloxy)-1,2,3,4-tetrahydrocyciopenta[b]indol-3-yl)essigsäure (Verbindung 1) oder ein pharmazeutisch akzeptables Salz, Hydrat oder Solvat davon
zur Verwendung nach einem der Ansprüche 1-6, wobei die Person nicht an Morbus Crohn leidet.

8. (R)-2-(7-(4-Cyclopentyl-3-(trifluormethyl)benzyloxy)-1,2,3,4-Tetrahydrocyclopenta[b]indol-3-yl)essigsäure (Verbindung 1) oder ein pharmazeutisch akzeptables Salz, Hydrat oder Solvat davon
zur Verwendung nach einem der Ansprüche 1-7,
wobei die therapeutisch wirksame Menge der Verbindung 1 oder des pharmazeutisch akzeptablen Salzes, Hydrats oder Solvats davon eine Menge ist, die etwa 1 mg bis etwa 5 mg der Verbindung 1 äquivalent ist.

9. (R)-2-(7-(4-Cyclopentyl-3-(trifluormethyl)benzyloxy)-1,2,3,4-tetrahydrocyclopenta[b]indol-3-yl)essigsäure (Verbindung 1),
oder ein pharmazeutisch akzeptables Salz, Hydrat oder Solvat davon,
zur Verwendung nach einem der Ansprüche 1-7
wobei die therapeutisch wirksame Menge der Verbindung 1 oder des pharmazeutisch akzeptablen Salzes, Hydrats oder Solvats davon eine Menge ist, die etwa 1 mg bis etwa 2 mg äquivalent ist.

10. (R)-2-(7-(4-Cyclopentyl-3-(trifluormethyl)benzyloxy)-1,2,3,4-tetrahydrocyclopenta[b]indol-3-yl)essigsäure (Verbindung 1),
oder ein pharmazeutisch akzeptables Salz, Hydrat oder Solvat davon
zur Verwendung nach einem der Ansprüche 1-7
wobei die therapeutisch wirksame Menge der Verbindung 1 oder des pharmazeutisch akzeptablen Salzes, Hydrats oder Solvats davon eine Menge ist, die etwa 1 mg der Verbindung 1 äquivalent ist.

11. (R)-2-(7-(4-Cyclopentyl-3-(trifluormethyl)benzyloxy)-1,2,3,4-tetrahydrocyclopenta[b]indol-3-yl)essigsäure (Verbindung 1),
oder ein pharmazeutisch akzeptables Salz, Hydrat oder Solvat davon
zur Verwendung nach einem der Ansprüche 1-7
wobei die therapeutisch wirksame Menge der Verbindung 1 oder des pharmazeutisch akzeptablen Salzes, Hydrats oder Solvats davon eine Menge ist, die etwa 2 mg der Verbindung 1 äquivalent ist.

12. (R)-2-(7-(4-Cyclopentyl-3-(trifluormethyl)benzyloxy)-1,2,3,4-tetrahydrocyclopenta[b]indol-3-yl)essigsäure (Verbindung 1), oder ein pharmazeutisch akzeptables Salz, Hydrat oder Solvat davon
zur Verwendung nach einem der Ansprüche 1-7
wobei die therapeutisch wirksame Menge der Verbindung 1 oder des pharmazeutisch akzeptablen Salzes, Hydrats oder Solvats davon eine Menge ist, die etwa 3 mg der Verbindung 1 äquivalent ist.

13. (R)-2-(7-(4-Cyclopentyl-3-(trifluormethyl)benzyloxy)-1,2,3,4-tetrahydrocyclopenta[b]indol-3-yl)essigsäure (Verbindung 1),
oder ein pharmazeutisch akzeptables Salz, Hydrat oder Solvat davon
zur Verwendung nach einem der Ansprüche 1-12
wobei die Verbindung 1 oder das pharmazeutisch verträgliche Salz, Hydrat oder Solvat davon ausgewählt ist aus: Verbindung 1, einem Calciumsalz von Verbindung 1 und einem L-Argininsalz von Verbindung 1.

14. (R)-2-(7-(4-Cyclopentyl-3-(trifiuoromethyl)benzyloxy)-1,2,3,4-tetrahydrocyclopenta[b]indol-3-yl)essigsäure (Verbindung 1)
oder ein pharmazeutisch akzeptables Salz, Hydrat oder Solvat davon
zur Verwendung nach einem der Ansprüche 1-12,
wobei die Verbindung 1 oder das pharmazeutisch verträgliche Salz, Hydrat oder Solvat davon ein L-Arginin-Salz der Verbindung 1 ist.

15. (R)-2-(7-(4-Cyclopentyl-3-(trifiuoromethyl)benzyloxy)-1,2,3,4-tetrahydrocyclopenta[b]indol-3-yl)essigsäure (Verbindung 1),
oder ein pharmazeutisch akzeptables Salz, Hydrat oder Solvat davon
zur Verwendung nach einem der Ansprüche 1-14
wobei die Verbindung 1 oder das pharmazeutisch annehmbare Salz, Hydrat oder Solvat davon eine wasserfreie, nicht-solvatisierte kristalline Form des L-Arginin-Salzes der Verbindung 1 ist.

16. (R)-2-(7-(4-Cyclopentyl-3-(trifluormethyl)benzyloxy)-1,2,3,4-tetrahydrocyclopenta[b]indol-3-yl)essigsäure (Verbindung 1), oder ein pharmazeutisch akzeptables Salz, Hydrat oder Solvat davon
zur Verwendung nach einem der Ansprüche 1-14
wobei die Verbindung 1 oder das pharmazeutisch annehmbare Salz, Hydrat oder Solvat davon ein kristalliner Free-Plate-Habitus des nicht-solvatisierten L-Arginin-Salzes der Verbindung 1 ist.

17. (R)-2-(7-(4-Cyclopentyl-3-(trifluormethyl)benzyloxy)-1,2,3,4-tetrahydrocyclopenta[b]indol-3-yl)essigsäure (Verbindung 1), oder ein pharmazeutisch akzeptables Salz, Hydrat oder Solvat davon
zur Verwendung nach einem der Ansprüche 1-12, wobei die Verbindung 1 oder das pharmazeutisch verträgliche Salz, Hydrat oder Solvat davon eine wasserfreie, nicht-solvatisierte kristalline Form der Verbindung 1 ist.

## Revendications

1. Acide (R)-2-(7-(4-cyclopentyl-3-(trifluorométhyl)benzyloxy)-1,2,3,4-tétrahydrocyclopenta[b]indol-3-yl)acétique (composé 1), ou un sel, hydrate ou solvate pharmaceutiquement acceptables de celui-ci, pour son utilisation dans un procédé de traitement et/ou de prévention d'une affection chez un individu le nécessitant,
le procédé comprenant l'administration à l'individu d'une quantité thérapeutiquement efficace du composé 1, ou du sel, hydrate, ou solvate pharmaceutiquement acceptables de celui-ci,
dans lequel l'affection est sélectionnée parmi les suivantes : sclérodermie systémique ; sclérodermie systémique diffuse ; sclérodermie systémique limitée ; sclérose systémique ; sclérose systémique diffuse ; sclérose systémique limitée ; sclérodermie ; sclérodermie diffuse ; sclérodermie limitée ; syndrome de CREST ; sclérose systémique cutanée diffuse ; sclérose systémique cutanée limitée ; sclérodermie cutanée diffuse ; sclérodermie cutanée limitée ; sclérodermie localisée ; morphée ; sclérodermie linéaire ; sclérodermie en coup de sabre ; sclérodermie sans atteinte cutanée ; sclérose systémique progressive ; sclérodermie progressive ; sclérose systémique avec maladie pulmonaire interstitielle associée ; sclérodermie avec maladie pulmonaire interstitielle associée ; sclérose systémique avec alvéolite ; sclérodermie avec alvéolite ; alvéolite associée à une sclérose systémique ; alvéolite associée à une sclérodermie ; sclérose systémique avec fibrose pulmonaire ; sclérodermie avec fibrose pulmonaire ; fibrose pulmonaire associée à une sclérose systémique ; fibrose pulmonaire associée à une sclérodermie ; sclérose systémique avec fibrose pulmonaire ; sclérodermie avec fibrose pulmonaire ; fibrose pulmonaire associée à une sclérose systémique ; fibrose pulmonaire associée à une sclérodermie ; crise rénale associée à une sclérose systémique ; crise rénale associée à une sclérodermie ; sclérodermie diffuse avec présence d'alvéolite fibrosante ; et sclérose systémique avec atteinte cutanée diffuse.

2. Acide (*R*)-2-(7-(4-cyclopentyl-3-(trifluorométhyl)benzyloxy)-1,2,3,4-tétrahydrocyclopenta[b]indol-3-yl)acétique (composé 1), ou un sel, hydrate ou solvate pharmaceutiquement acceptables de celui-ci,
pour son utilisation selon la revendication 1,
dans lequel l'affection est une sclérose systémique.

3. Acide (*R*)-2-(7-(4-cyclopentyl-3-(trifluorométhyl)benzyloxy)-1,2,3,4-tétrahydrocyclopenta[b]indol-3-yl)acétique (composé 1), ou un sel, hydrate ou solvate pharmaceutiquement acceptables de celui-ci,
pour son utilisation selon la revendication 1,
dans lequel l'affection est une sclérodermie.

4. Acide (*R*)-2-(7-(4-cyclopentyl-3-(trifluorométhyl)benzyloxy)-1,2,3,4-tétrahydrocyclopenta[b]indol-3-yl)acétique (composé 1), ou un sel, hydrate ou solvate pharmaceutiquement acceptables de celui-ci,
pour son utilisation selon la revendication 1,
dans lequel l'affection est un syndrome de CREST.

5. Acide (*R*)-2-(7-(4-cyclopentyl-3-(trifluorométhyl)benzyloxy)-1,2,3,4-tétrahydrocyclopenta[b]indol-3-yl)acétique (composé 1), ou un sel, hydrate ou solvate pharmaceutiquement acceptables de celui-ci,
pour son utilisation selon l'une des revendications 1 à 4,
dans lequel l'individu n'a pas d'affection abdominale inflammatoire ou de syndrome du côlon irritable.

6. Acide (*R*)-2-(7-(4-cyclopentyl-3-(trifluorométhyl)benzyloxy)-1,2,3,4-tétrahydrocyclopenta[b]indol-3-yl)acétique (composé 1), ou un sel, hydrate ou solvate pharmaceutiquement acceptables de celui-ci,
pour son utilisation selon l'une des revendications 1 à 5,
dans lequel l'individu n'a pas de rectocolite hémorragique.

7. Acide (*R*)-2-(7-(4-cyclopentyl-3-(trifluorométhyl)benzyloxy)-1,2,3,4-tétrahydrocyclopenta[b]indol-3-yl)acétique (composé 1), ou un sel, hydrate ou solvate pharmaceutiquement acceptables de celui-ci,
pour son utilisation selon l'une des revendications 1 à 6,
dans lequel l'individu n'a pas la maladie de Crohn.

8. Acide (*R*)-2-(7-(4-cyclopentyl-3-(trifluorométhyl)benzyloxy)-1,2,3,4-tétrahydrocyclopenta[b]indol-3-yl)acétique (composé 1), ou un sel, hydrate ou solvate pharmaceutiquement acceptables de celui-ci,
pour son utilisation selon l'une des revendications 1 à 7,
dans lequel la quantité thérapeutiquement efficace du composé 1, ou du sel, hydrate, ou solvate pharmaceutiquement acceptables de celui-ci, est une quantité équivalente à environ 1 mg à environ 5 mg de composé 1.

9. Acide (*R*)-2-(7-(4-cyclopentyl-3-(trifluorométhyl)benzyloxy)-1,2,3,4-tétrahydrocyclopenta[b]indol-3-yl)acétique (composé 1), ou un sel, hydrate ou solvate pharmaceutiquement acceptables de celui-ci,
pour son utilisation selon l'une des revendications 1 à 7,
dans lequel la quantité thérapeutiquement efficace du composé 1, ou du sel, hydrate, ou solvate pharmaceutiquement acceptables de celui-ci, est une quantité équivalente à environ 1 mg à environ 2 mg.

10. Acide (*R*)-2-(7-(4-cyclopentyl-3-(trifluorométhyl)benzyloxy)-1,2,3,4-tétrahydrocyclopenta[b]indol-3-yl)acétique (composé 1), ou un sel, hydrate ou solvate pharmaceutiquement acceptables de celui-ci,
pour son utilisation selon l'une des revendications 1 à 7,
dans lequel la quantité thérapeutiquement efficace du composé 1, ou du sel, hydrate, ou solvate pharmaceutiquement acceptables de celui-ci, est une quantité équivalente à environ 1 mg de composé 1.

11. Acide (*R*)-2-(7-(4-cyclopentyl-3-(trifluorométhyl)benzyloxy)-1,2,3,4-tétrahydrocyclopenta[b]indol-3-yl)acétique (composé 1), ou un sel, hydrate ou solvate pharmaceutiquement acceptables de celui-ci,
pour son utilisation selon l'une des revendications 1 à 7,
dans lequel la quantité thérapeutiquement efficace du composé 1, ou du sel, hydrate, ou solvate pharmaceutiquement acceptables de celui-ci, est une quantité équivalente à environ 2 mg de composé 1.

12. Acide (R)-2-(7-(4-cyclopentyl-3-(trifluorométhyl)benzyloxy)-1,2,3,4-tétrahydrocyclopenta[b]indol-3-yl)acétique (composé 1), ou un sel, hydrate ou solvate pharmaceutiquement acceptables de celui-ci,
pour son utilisation selon l'une des revendications 1 à 7,
dans lequel la quantité thérapeutiquement efficace du composé 1, ou du sel, hydrate, ou solvate pharmaceutiquement acceptables de celui-ci, est une quantité équivalente à environ 3 mg de composé 1.

13. Acide (*R*)-2-(7-(4-cyclopentyl-3-(trifluorométhyl)benzyloxy)-1,2,3,4-tétrahydrocyclopenta[b]indol-3-yl)acétique (composé 1), ou un sel, hydrate ou solvate pharmaceutiquement acceptables de celui-ci,
pour son utilisation selon l'une des revendications 1 à 12,
dans lequel le composé 1, ou le sel, hydrate, ou solvate pharmaceutiquement acceptables de celui-ci, est sélectionné parmi : le composé 1, un sel de calcium du composé 1, et un sel de L-arginine du composé 1.

14. Acide (*R*)-2-(7-(4-cyclopentyl-3-(trifluorométhyl)benzyloxy)-1,2,3,4-tétrahydrocyclopenta[b]indol-3-yl)acétique (composé 1), ou un sel, hydrate ou solvate pharmaceutiquement acceptables de celui-ci,
pour son utilisation selon l'une des revendications 1 à 12,
dans lequel le composé 1, ou le sel, hydrate, ou solvate pharmaceutiquement acceptables de celui-ci, est un sel de L-arginine du composé 1.

15. Acide (*R*)-2-(7-(4-cyclopentyl-3-(trifluorométhyl)benzyloxy)-1,2,3,4-tétrahydrocyclopenta[b]indol-3-yl)acétique (composé 1), ou un sel, hydrate ou solvate pharmaceutiquement acceptables de celui-ci,
pour son utilisation selon l'une des revendications 1 à 14,
dans lequel le composé 1, ou le sel, hydrate, ou solvate pharmaceutiquement acceptables de celui-ci, est une forme cristalline, anhydre, non solvatée du sel de L-arginine du composé 1.

16. Acide (*R*)-2-(7-(4-cyclopentyl-3-(trifluorométhyl)benzyloxy)-1,2,3,4-tétrahydrocyclopenta[b]indol-3-yl)acétique (composé 1), ou un sel, hydrate ou solvate pharmaceutiquement acceptables de celui-ci,
pour son utilisation selon l'une des revendications 1 à 14,
dans lequel le composé 1, ou le sel, hydrate, ou solvate pharmaceutiquement acceptables de celui-ci, est un habitus cristallin exempt de plaque du sel non solvaté de L-arginine du composé 1.

17. Acide (*R*)-2-(7-(4-cyclopentyl-3-(trifluorométhyl)benzyloxy)-1,2,3,4-tétrahydrocyclopenta[b]indol-3-yl)acétique (composé 1), ou un sel, hydrate ou solvate pharmaceutiquement acceptables de celui-ci,
pour son utilisation selon l'une des revendications 1 à 12,
dans lequel le composé 1, ou le sel, hydrate, ou solvate pharmaceutiquement acceptables de celui-ci, est une forme cristalline, anhydre, non solvatée du composé 1.
